(19) Europäisches Patentamt
European Patent Office
Office européen des brevets

(11) Numéro de publication: **0 126 013**
**B1**

(12) **FASCICULE DE BREVET EUROPÉEN**

(45) Date de publication du fascicule du brevet: **23.09.87**

(51) Int. Cl.⁴: **C 07 D 295/18**, A 61 K 31/395

(21) Numéro de dépôt: **84401007.4**

(22) Date de dépôt: **17.05.84**

(54) **Dithiodiacétamides cycliques, procédé pour leur préparation et compositions pharmaceutiques en contenant.**

(30) Priorité: **17.05.83 FR 8308155**

(43) Date de publication de la demande: **21.11.84 Bulletin 84/47**

(45) Mention de la délivrance du brevet: **23.09.87 Bulletin 87/39**

(84) Etats contractants désignés: **AT BE CH DE FR GB IT LI LU NL SE**

(56) Documents cité: **Néant**

(73) Titulaire: **SANOFI, société anonyme, 40, Avenue George V, F-75008 Paris (FR)**
Titulaire: **INSTITUT NATIONAL DE LA SANTE ET DE LA RECHERCHE MEDICALE (INSERM), 101, rue de Tolbiac, F-75654 Paris Cedex 13 (FR)**

(72) Inventeur: **Rosa, Jean, 7, rue Vergniaud, F-75013 Paris (FR)**
Inventeur: **Castaigne, Jean- Paul, 3, rue Largillière, F-75016 Paris (FR)**
Inventeur: **Demarne, Henri, Le Florence Avenue Major Flandre, F-34000 Montpellier (FR)**
Inventeur: **Tozzolino, Pierre, Lotissement Coubet Serres- Morlaas, F-64160 Morlaas (FR)**

(74) Mandataire: **Gillard, Marie- Louise, Cabinet Beau de Loménie 55, Rue d'Amsterdam, F-75008 Paris (FR)**

**EP 0 126 013 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1987

**Description**

La présente invention à pour objet das dithiodiacétamides cycliques, un procédé pour leur préparation et des compositions pharmaceutiques pour l'inhibition de la malformation ou de la destruction des érythrocytes due à une modification génétique de l'hémoglobine ou à des parasites et donc utiles dans le traitement de la drépanocytose et du paludisme.

Il est connu que la drépanocytose est une maladie génétique qui comporte une anomalie de la structure de l'hémoglobine, dont l'acide aminé Glu-6 de la chaîne bêta est remplacé par l'acide aminé Val pour donner l'hémoglobine S qui polymérise.

Ladite polymérisation, lors de la désoxygénation du globule rouge entraîne la falciformation de ce dernier qui devient rigide, circule mal et se bloque dans les petits vaisseaux. Les individus porteurs de deux gènes drépanocytaires sont ainsi sous la menace permanente d'une complication fatale.

Certains disulfures ont été décrits dans la littérature comme inhibant la falciformation; notamment la cystamine a été indiquée comme particulièrement active ("Developments of Therapeutic Agents for Sikle Cell Disease", Inserm Symposium, 1979, North Holland : Amsterdam, Editeurs J. Rosa Y. Beuzard J. Hercules; pages 139-153).

Il a été maintenant trouvé que les dithiodiacétamides cycliques de formule I

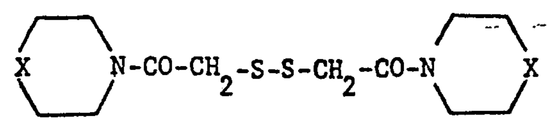

I

dans laquelle X représente une liaison directe, un atome d'oxygène ou de soufre, un groupe méthylène, un groupe éthylène ou un groupe N-R, où R est un atome d'hydrogène ou un groupe alkyle inférieur, (hydroxy)alkyle inférieur, alcanoyle inférieur, ou un groupe phényle, sont très actifs dans l'inhibition de la polymérisation de l'hémoglobIne S et de la falciformation des globules rouges.

Il a été également trouvé d'une façon surprenante que les dithiodiacétamides cycliques de formule I ci-dessus sont actifs sur les parasites affectant les érythrocytes, tels que les Plasmodia et les Babesiae. Plus particulièrement, ils montrent une activité schizonticide in vitro sur "Plasmodium falciparum" et in vivo sur "Plasmodium berghei".

L'expression "inhibition de la malformation ou de la destruction des érythrocytes", telle qu'utilisée ici, définit une inhibition qui peut être soit directe sur l'hémoglobine soit indirecte par l'inhibition de la croissance des parasites dans le globule rouge.

Ainsi, la présente invention à pour objet, selon un de ses aspects, les dithiodiacétamides cycliques de formule I ci-dessus ainsi que leurs sels pharmacautiquement acceptables.

Le terme "alkyle inférieur", tel qu'utilisé ici, désigne les groupes méthyle, éthyle, propyle et isopropyle.

Le terme "alcanoyle inférieur", désigne les groupes formyle, acétyle et propionyle.

Le présente invention a également pour objet, selon un autre de ses aspects, un procédé pour la préparation des dithiodiacétamides cycliques de formule I ci-dessus caractérisé en ce que l'on traite un dithiodiacétate ou un mercaptoacétate d'alkyle inférieur, de méthyle ou d'éthyle de préférence, avec une amine secondaire cyclique de formule

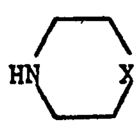

II

dans laquelle X est tel que défini ci-dessus à une température de 15 à 60°C et, lorsqu'on utilise un mercaptoacétate d'alkyle inférieur comme produit de départ, on soumet le produit ainsi obtenu à une oxydation; et l'on transforme éventuellement le produit ainsi obtenu dans ses sels pharmaceutiquement acceptables.

La transamination avec l'amine II peut être effectuée en mélangeant les réactifs sans utiliser de solvants, ou bien dans un solvant, de préférence un alcool, plus particulièrement le même alcool estérifiant l'acide dithiodiacétique dans le produit de départ.

Après une période de 6 à 24 heures, dépendant de la température, le produit final est isolé selon les techniques habituelles, par exemple par évaporation et purification.

Lorsque, comme produit de départ on utilise un mercaptoacétate d'alkyle inférieur, le produit ainsi obtenu de formule

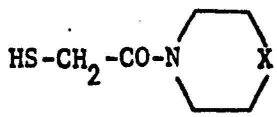

III

dans laquelle X est tel que défini ci-dessus est soumis à une oxydation en le laissant s'oxyder à l'air éventuellement enrichie d'oxygène, dans une solution aqueuse ou en l'absence de solvants, ou bien en utilisant un agent oxydant tel que le brome, l'iode ou l'eau oxygénée.

Selon un mode opératoire préféré, on utilise une solution à 15 % d'eau oxygénée à la température de 10-30° C. Le dithiodiacétamide cyclique qui se forme précipite et est isolé par simple filtration ou bien il est isolé par évaporation, à pression réduite de préférence, de l'eau.

Les dithiodiacétamides cycliques de la présente invention possèdent une activité biologique remarquable car ils empêchent la malformation ou la destruction des érythrocytes due à la drépanocytose ou à des parasites, tels que les Plasmodia et les Babesiae. Plus particulièrement, les dithiodiacétamides cycliques de formule I ci-dessus inhibent la falciformation des érythrocytes des drépanocytaires.

L'inhibition de la falciformation des érythrocytes humains a été évaluée selon la méthode consistant à laver et incuber, dans un tampon salin (pH 7,40 - 0,15 M), les globules rouges des drépanocytaires pendant une heure à 37° C dans un bain-marie sous agitation circulaire en présence du produit ci-dessus à différentes concentrations. Dans cette opération le rapport molaire produit/hémoglobine est testé initialement dans l'intervalle allant de 0,5 à 20. En fonction des résultats obtenus, ce rapport est modifié. A la fin de l'incubation l'excès de produit est enlevé par lavage. La suspension cellulaire ajustée à un hématocrite de 5 % est transférée dans un Erlenmeyer et incubée à 37° C sous un courant d'un mélange humidifié d'azote et d'oxygène. La concentration d'oxygène est réglée par une pompe à mélange gazeux. Le gaz effluent est transféré dans un autre tube contenent du formaldéhyde. A la fin de l'incubation la suspension cellulaire est transférée dans le formaldéhyde par simple retournement de la fiole.

La proportion des cellules déformées et de celles ayant les caractéristiques de drépanocytes (ayant deux prolongements filiformes) est évaluée à l'aide d'un microscope ayent une optique interférentielle de NOMARSKY. L'inhibition de la falciformation a été calculée selon la formule suivante :

$$\frac{\text{\% cellules falciformées - témoins} \quad \text{\% cellules falciformées en présence de la drogue}}{\text{\% cellules falciformées du témoin}}$$

Dans ce test, un composé représentatif de la présente invention, la 4,4'-dithiodiecéthyldimorpholine (formule I X = 0), a montré une inhibition de 75 % de le falciformation.

Dans les mêmes conditions la cystamine donne une inhibition de 33 %.

Les composés de la présente invention sont peu toxiques et peuvent être utilisés ccmme médicements.

Ainsi, selon un autre de ses aspects, la présente invention se réfère à des compositions pharmaceutiques pour l'inhibition de la malformation ou de la destruction des érythrocytes due à une modification génétique de l'hémoglobine ou à des parasites renfermant, en tant qu'ingrédients actifs, des dithiodiacétamides cycliques de formule I ci-dessus, ainsi que leurs sels d'addition éventuels pharmaceutiquement acceptables.

Dans les compositions pharmaceutiques de la présente invention pour l'administration orale, sublinguale, sous-cutanée, intramusculaire, intraveineuse, transdermique ou rectale, les ingrédients actifs de formule I ci-dessus peuvent être administrés sous forme unitaires d'administration en mélange avec des supports pharmaceutiques classiques, aux animaux et aux êtres humains pour le traitement de la drépanocytose, du paludisme ou de la babébiose. Parmi les formes unitaires d'administration appropriées, il y a les formes par voie orale, telles que les comprimés, les gélules, les poudres, les granules et les solutions ou suspensions orales et les formes d'administration sublinguale et buccale, de même que formes d'administration sous-cutanée, intramusculaire ou intraveineuse, éventuellement par perfusion et rectale.

Afin d'obtenir l'effet désiré la dose de principe actif peut varier entre 0,1 et 100 mg par kg de poids du corps et par jour.

Chaque dose unitaire peut contenir de 10 à 1000 mg d'ingrédient actif en combinaison avec un support pharmaceutique. Cette dose unitaire peut être administrée 1 à 4 fois par jour, éventuellement par perfusion pour le traitement par exemple de la drépanocytose, du paludisme et de la babébiose.

On prépare simplement les poudres pour l'administration orale en broyant le composé actif à une finesse convenable et en mélangeant avec un diluant broyé similairement. Le diluant peut être un matériau à base de glucide comestible, tel que l'amidon. Il est avantageux qu'un agent édulcorant ou un sucre, ainsi qu'une huile aromatisante, soient présents.

On prépare les granulés destinés à la reconstitution d'une préparation liquide orale en utilisant des diluants solubles dans l'eau; on mouille le composé actif et un diluant soluble dans l'eau, tel que le saccharose, le glucose, etc., avec un liant, tel que le mucilage d'acacia, une solution de gélatine, une solution de méthylcellulose, et on fait passer sur un tamis en forçant pour former des granulés que l'on fait sécher. Il est avantageux d'introduire dans la composition un agent de mise en suspension comme la gomme adragante.

On fait les gélules en préparant un mélange pulvérulent comme il est décrit ci-dessus et en le mettant dans des gaines de gélatine mises en forme. Comme adjuvant à l'opération de remplissage il est avantageux d'ajouter un lubrifiant tel que le talc, le stéarate de magnésium et le stéarate de calcium au mélange pulvérulent avant l'opération de remplissage.

On fait les comprimés en préparant un mélange pulvérulent en granulant ou en tronçonnant, en ajoutant un lubrifiant et en comprimant pour former des comprimés. On prépare le mélange pulvérulent en mélangeant le composé désiré, convenablement broyé, avec un diluant ou une base telle que l'amidon, le saccharose, le kaolin, le phosphate bicalcique, etc. On peut granuler le mélange pulvérulent en mouillant avec le liant, tel que sirop pâte d'amidon ou mucilage d'acacia et en forçant à travers un tamis. Comme méthode autre que la granulation, on peut tronçonner le mélange pulvérulent, c'est-à-dire, le faire passer à travers la machine à comprimés et casser les comprimés résultent imparfaitement formés en fragments (tronçons). On peut lubrifier les tronçons pour les empêcher de coller aux comprimés en formant des cubes, en ajoutant un sel stéarate, du talc ou une huile minérale. On comprime ensuite le mélange lubrifiant pour constituer des comprimés.

Les comprimés peuvent être avantageusement munis d'un revêtement protecteur constitué d'un enduit de scellement de gomme-laque un revêtement de sucre et de méthylcellulose, et un enduit vernis de cire de carnaubs, ou encore on peut le traiter d'une autre manière de telle sorte qu'ils aient une activité prolongée ou retardée et qu'ils libèrent continuellement une quantité prédéterminée de principe actif.

On prépare les fluides pour administration orale sous des formes posologiques unitaires, telles que les sirops où chaque cuillère à café de composition contient une quantité prédéterminée du composé actif à administrer.

Une préparation sous forme de sirop ou d'élixir peut contenir l'ingrédient actif conjointement avec un édulcorant, du Methylparaben et du Propylparaben comme antiseptiques, ainsi qu'un agent donnant du goût et un colorant approprié.

Pour une application rectale, on a recours à des suppositoires qui sont préparés avec des liants fondant à la température rectale, par exemple du beurre de cacao ou des polyéthylèneglycols.

Pour une application parentérale, on utilise des suspensions aqueuses des solutions salines, isotoniques ou des solutions stériles et injectables qui contiennent des agents de dispersion et/ou mouillants pharmacologiquement compatibles, par exemple le propylèneglycol ou le butylèneglycol.

Le principe actif peut être formulé également sous forme de microcapsules, éventuellement avec un ou plusieurs supports ou additifs.

Les exemples suivants illustrent l'invention sans toutefois la limiter.

**Exemple 1**

A. On introduit 0,1 mol de thioglycolate de méthyle dans une fiole Erlenmeyer munie d'un agitateur magnétique, puis on ajoute 0,11 mol de pyrrolidine et on maintient l'agitation 6-12 heures à la température ambiante. A la fin de la réaction on élimine la pyrrolidine en excès et on soumet le produit obtenu à une distillation fractionnée sous pression réduite. On obtient ainsi la 1-mercapto-acétylpyrrolidine avec un rendement de 86 % après distillation.

En opérant comme décrit ci-dessus, mais en remplaçant la pyrrolidine par 0,11 mol de 1-méthylpipérazine et de morpholine, on obtient respectivement:

- la 1-méthyl-4-mercaptoacétylpipérazine et
- la 4-mercaptoacétylmorpholine.

B. On introduit 0,1 mol de 1-mercaptoacétylpyrrolidine dans une fiole Erlenmeyer munie d'un agitateur magnétique. On additionne 0,1 mol d'eau oxygénée (à 15 %) en maintenant la température du milieu entre 10 et 15°C et on agite ensuite le milieu pendant 2 heures à la température ambiante. Par refroidissement dans un bain de glace, les cristaux de disulfure apparaissent. Le produit est filtré, séché et éventuellement recristallisé dans l'eau ou dans un mélange eau/éthanol. On obtient ainsi avec un rendement de 90 % la 1,1'-dithiodiacétyldipyrrolidine (formule I X = liaison directe); p.f. 79°C (Kofler).

En opérant comme décrit ci-dessus, à partir de la 1-méthyl-4-mercaptoacétylpipérazine et de la 4-mercaptoacétylmorpholine on obtient respectivement:

- la 4,4'-dithiodiacétyldi-(1-méthyl)pipérazine (formule I X = N-$CH_3$); p.f. 110°C (Kofler); et
- la 4,4'-dithiodiacétyldimorpholine (formule I, X = oxygène) sous forme de liquide visqueux.

**Exemple 2**

A une solution de 47,6 g de dithiodiacétate d'éthyle dans 150 ml d'éthanol on ajoute 34,8 g de morpholine. On chauffe le mélange ainsi obtenu à 50°C pendant 24 heures, puis on évapore l'éthanol et la morpholine en excès sous pression réduite. On obtient ainsi la 4,4'-dithiodiacétyldimorpholine sous forme de liquide visqueux.

De la même façon, en traitant 0,2 mol de dithiodiacétate d'éthyle avec, respectivement 0,4 mol de pyrrolidine et 0,4 mol de 1-méthylpipérazine dans de l'éthanol, on obtient, respectivement:

- la 1,1'-dithiodiacétyldipyrrolidine; p.f. 79°C (Kofler) et

- la 4,4'-dithiodiacétyldi-(1-méthyl)pipérazine; p.f. 110°C (Kofler).

**Exemple 3**

On prépare une composition pour comprimés renfermant en tant que principe actif un des composés décrits dans les exemples 1 et 2 ayant la formule suivante:

| | |
|---|---|
| principe actif | 500 mg |
| glycine | 120 mg |
| cellulose microgranulaire | 70 mg |
| silice précipitée | 18 mg |
| carboxyméthylamidon | 30 mg |
| stéarate de magnésium | 11 mg |
| talc | 11 mg |

On mélange les quantités calculées des composants pendant 30 minutes, puis on granule à sec et on fait passer le mélange à travers un tamis à mailles de 1,6 mm. On comprime ensuite en utilisant un poinçon ayant la forme d'un petit bâton. On obtient ainsi des comprimés pesant chacun 760 mg et renfermant chacun 500 mg de principe actif.

**Exemple 4**

On prépare des comprimés selon l'exemple 3. Les comprimés ainsi obtenus sont enrobés à l'aide d'une suspension de phtalate de dibutyle, de polyméthacrylate de butyle et diméthylaminoéthyle de polyéthylèneglycol 1500, de silice précipitée, de bioxyde de titane et de talc dans un mélange acétone: isopropanol 1:1 ayant une concentration en résidu sec de 10 % environ. On obtient ainsi des comprimés enrobés pesant chacun 780 mg et renfermant chacun 500 mg de principe actif.

**Exemple 5**

On prépare un granulé destiné à la reconstitution d'une préparation liquide orale renfermant, en tant que principe actif, un des composés décrits dans les exemples 1 et 2, ayant la composition suivante:

| | |
|---|---|
| principe actif | 3,60 g |
| saccharose | 50,00 g |
| carboxyméthylcellulose sodium | 0,80 g |
| acide citrique | 0,10 g |
| citrate trisodique | 0,90 g |
| benzoate de sodium | 0,25 g |
| saccharine sodique | 0,15 g |
| aromatisant | 0,50 g |

Le volume du granulé ainsi obtenu est porté à 100 ml avec de l'eau pour sirops. Une dose unitaire de 5 ml du sirop extemporané ainsi obtenu contient 180 mg de principe actif.

Pour préparer le granulé, on pulvérise les quantités calculées de tous les composants, sauf le saccharose, puis on mélange la poudre ainsi obtenue avec le saccharose jusqu'à obtenir un granulé homogène.

**Exemple 6**

On prépare selon l'exemple 5 un granulé destiné à la reconstitution d'une préparation liquide orale ayant la composition suivante:

| | |
|---|---|
| principe actif | 7,00 g |
| saccharose | 46,60 g |
| carboxyméthylcellulose sodium | 0,90 g |
| acide citrique | 0,10 g |
| citrate trisodique | 0,90 g |
| benzoate de sodium | 0,25 g |
| saccharine sodique | 0,15 g |
| aromatisant | 0,50 g |

Une dose mitaire de 5 ml du sirop extemporené ainsi obtenu contient 350 mg de principe actif.

**Exemple 7**

On prépare selon l'exemple 5 un granulé destiné à la reconstitution d'une préparation liquide orale ayant la composition suivante:

| | |
|---|---|
| principe actif | 8,00 g |
| saccharose | 45,60 g |
| carboxyméthylcellulose sodium | 1,00 g |
| acide citrique | 0,10 g |
| citrate trisodique | 0,90 g |
| benzoate de sodium | 0,25 g |
| saccharine sodique | 0,15 g |
| aromatisant | 0,50 g |

Une dose unitaire de 5 ml du sirop extemporané ainsi obtenu contient 400 mg de principe actif.

**Exemple 8**

On prépare des comprimés à base d'un des composés décrits dans les exemples 1 et 2 ayant le composition suivante :

| | |
|---|---|
| principe actif | 350 mg |
| cellulose microcristalline | 100 mg |
| lactose | 125 mg |
| stéarate de magnésium | 10 mg |
| talc | 15 mg |

On fait passer les poudres à travers un tamis de 0,3 mm, puis on mélange les ingrédients jusqu'à ce qu'on obtienne un mélange homogène qui est comprimé et granulé. Les granules ainsi obtenus sont utilisés pour former des comprimés par compression.

Poids d'un comprimé : 600 mg.

**Exemple 9**

Comprimés à base d'un des composés décrits dans les exemples 1 et 2 ayant la composition suivante:

| | |
|---|---|
| principe actif | 150 mg |
| cellulose microcristalline | 75 mg |
| talc | 15 mg |
| polyvinylpyrrolidone | 30 mg |
| silice précipitée | 25 mg |
| stéarate de magnésium | 5 mg |

On mélange intimement dans une mélangeuse-pétrisseuse tous les ingrédients, sauf le lubrifiant, pendant 15 minutes, puis on pétrit par addition greduelle d'eau. On fait passer la masse à travers un tamis de 1,25 mm. On sèche le granulé dans une étuve à ventilation forcée jusqu'à ce qu'on obtienne une humidité résiduelle relativement réduite (environ 2 %). On uniformise le granulé, on ajoute le lubrifiant et on forme des comprimés par compression. Poids d'un comprimé : 300 mg.

De la même façon on prépare des comprimés contenant 250 mg de principe actif.

**Exemple 10**

On prépare des comprimés enrobés à base d'un des composés décrits dans les exemples 1 et 2, ayant la composition suivante:

| | |
|---|---|
| principe actif | 150 mg |
| carboxyméthylamidon | 10 mg |
| cellulose microcristalline | 85 mg |
| lactose | 135 mg |
| huile de ricin hydrogénée | 10 mg |
| stéarate de magnésium | 5 mg |

en opérant comme décrit à l'exemple 9. On recouvre les comprimés ainsi obtenus par un revêtement ayant la composition suivante:

| | |
|---|---|
| phtalate de butyle | 0,300 mg |
| polyméthacrylate de butyle et diméthylaminoéthyle | 1,850 mg |
| polyéthylèneglycol 1500 | 0,080 mg |
| silice précipitée | 0,020 mg |
| talc | 0,900 mg |
| bioxyde de titane | 1,850 mg |

dissous dans un solvant qui est élimité par évaporation dans une étuve à ventilation forcée.

Poids d'un comprimé : 400 mg.

**Exemple 11**

Suppositoires à base d'un des composés décrits dans les exemples 1 et 2 ayant la composition suivante:

| | |
|---|---|
| principe actif | 300 mg |
| masse pour suppositoires | 1.450 mg |

On met en suspension la substance active finement pulvérisée dans la masse pour suppositoires à 37° C et on verse le mélange dans des moules légèrement refroidis au préalable.

Poids d'un suppositoire : 1.750 mg.

**Revendications** pour les etats contractants BE, CH, DE, FR, GB, IT, LI, LU, NL, SE.

Dithiodiacétamide cyclique de formule

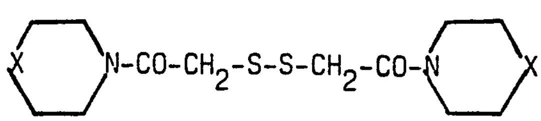

dans laquelle X représente une liaison directe, un atome d'oxygène ou de soufre, un groupe méthylène, un groupe éthylène ou un groupe N-R, où R est un atome d'hydrogène ou un groupe alkyle inférieur, (hydroxy) alkyle inférieur, formyle, acétyle, propinyle ou un groupe phényle ou un de ses sels éventuels pharmaceutiquement acceptables.

Le terme alkyle inférieur désigne les groupes méthyle, éthyle, propyle et isopopyle.

2. 4,4'-dithiodiacétyldimorpholine,

3. 1,1'-dithiodiacétyldipyrrolidine,

4. 4,4'-dithiodiacétyldi-(1-méthyl)pipérazine.

5. Procédé pour la préparation d'un composé selon la revendication 1, caractérisé en de que l'on traite un dithiodiacétate ou un mercaptoacétate d'alkyle inférieur avec une amine de formule

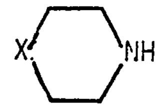

dans laquelle X est tel que défini dans la revendication 1, à une température de 15 à 60° C et, lorsqu'on utilise un mercaptoacétate d'alkyle inférieur comme produit de départ, on soumet le produit ainsi obtenu à une oxydation; et l'on transforme éventuellement le produit ainsi obtenu en ses sels phamarceutiquement acceptables. Le terme alkyle inférieur désigne les groupe méthyle, éthyle, propyle et isopyle.

6. Composition pharmaceutique pour l'inhibition de la malformation ou de la destruction des érythrocytes due à une modification génétique de l'hémoglobine ou à des parasites, caractérisée en ce qu'elle contient, en tant que principe actif, un composé selon l'une quelconque des revendications 1 à 4.

7. Composition selon la revendication 6, caractérisée en ce qu'elle est sous forme d'unité de dosage.

8. Composition selon la revendication 7, caractérisée en ce que chaque unité de dosage contient de 10 à 100 mg de principe actif.

**Revendications** pour l'état contractant : AT

1. Procédé pour l'obtention de dithiodiacétamide cyclique de formule

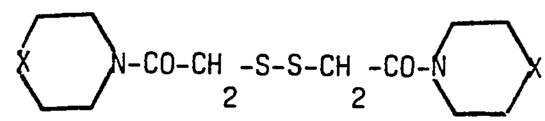

dans laquelle X représente une liaison directe, un atome d'oxygène ou de soufre, un groupe méthylène, un groupe éthylène ou un groupe N-R, où R est un atome d'hydrogène ou un groupe alkyle inférieur, (hydroxy) alkyle inférieur, formyle, acétyle, propionyle ou un groupe phényle ou un de ses sels éventuels pharmaceutiquement acceptables, caractérisé en ce qu'il consiste:

1) à traiter un dithiodiacétate ou un mercaptoacétate d'alkyle inférieur avec une amine secondaire cyclique de formule

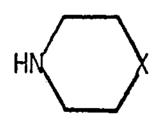

dans laquelle X est tel que défini ci-dessus à une température comprise entre 15 et 60° C,

2) à soumettre le produit obtenu à une oxydation lorsque le composé de départ est un mercaptoacétate d'alkyle inférieur;

3) à transformer éventuellement le produit obtenu à l'une des étapes 1) ou 2) en l'un de ses sels pharmaceutiquement acceptables.

Le terme alkyle inférieur désigne les groupes méthyle, éthyle, propyle et isopropyle.

2. Procédé selon la revendication 1, caractérisé en ce que l'on opére l'étape 1) dans un solvant.

3. Procédé selon la revendication 2, caractérisé en ce que le solvant est un alcool.

4. Procédé selon l'une quelconque des revendications 1 à 3, caractérisé en ce que le produit de départ est un mercaptoacétate d'alkyle inférieur qui donne par réaction avec une amine secondaire cyclique de formule II:

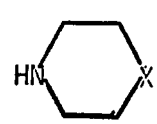

dans laquelle X est tel que défini dans la revendication 1 un composé de formule:

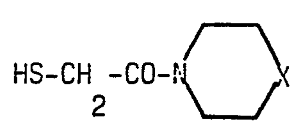

dans laquelle X est tel que défini ci-dessus, ledit composé de formule III est ensuite soumis à une oxydation à l'air ou en présence d'un agent oxydant; tel que le brome, l'iode ou l'eau oxygénée pour donner le composé

de formule I.
Le terme alkyle inférieur désigne les groupes méthyle, éthyle, propyle et isopopyle.
5. Procédé selon la revendication 4, caractérisé en ce que l'on utilise comme agent oxydant une solution à 15 % d'eau oxygénée à la température de 10 à 30° C.

**Patentansprüche** für die Vertragsstaaten: BE, CH, DE, FR, GB, IT, LI, LU, NL, SE

1. Zyklisches Dithiodiacetamid der Formel

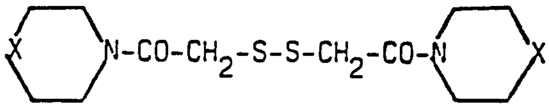

in der X eine direkte Bindung, Sauerstoff oder Schwefel, Methylen, Ethylen oder N-R ist, wobei R Wasserstoff oder niedriges Alkyl oder Hydroxyalkyl, Formyl, Acetyl, Propionyl oder Phenyl ist, der Ausdruck "niedriges Alkyl" bedeutet hier Methyl, Ethyl, Propyl oder Isopropyl,
oder eines seiner gegebenenfalls pharmazeutisch verträglichen Salze.
2. 4,4'-Dithiodiacetyldimorpholin.
3. 1,1'-Dithiodiacetyldipyrrolidin.
4. 4,4'-Dithiodiacetyldi-(1-methyl)-piperazin.
5. Verfahren zur Herstellung einer Verbindung nach Anspruch 1,
gekennzeichnet durch
Behandlung eines niedrigen Alkyldithiodiacetats oder -mercaptoacetat mit einem Amin der Formel

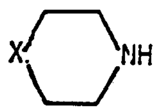

in der X wie in Anspruch 1 definiert ist, bei einer Temperatur von 15 bis 60° C und
bei Verwendung eines niedrigen Alkylmercaptoacetats als Ausgangsprodukt Oxidation des so erhaltenen Produkts und
gegebenenfalls Umwandlung des so erhaltenen Produkts in seine pharmazeutisch verträglichen Salze (der Ausdruck "niedriges Alkyl" bedeutet hier Methyl, Ethyl, Propyl und Isopropyl).
6. Pharmazeutische Zusammensetzung zur Hemmung der Missbildung oder Zerstörung von Erythrozyten, verursacht durch eine genetische Veränderung des Hämoglobins oder durch Parasiten,
gekennzeichnet durch
den Gehalt an einer Verbindung nach einem der Ansprüche 1 bis 4 als Wirkstoff.
7. Zusammensetzung nach Anspruch 6 in Form einer Dosiereinheit.
8. Zusammensetzung nach Anspruch 7,
dadurch gekennzeichnet,
daß jede Dosiereinheit 10 bis 100 mg Wirkstoff enthält.

**Patentansprüche** für den Vertragsstaat: AT

1. Verfahren zur Herstellung eines zyklischen Dithiodiacetamids der Formel

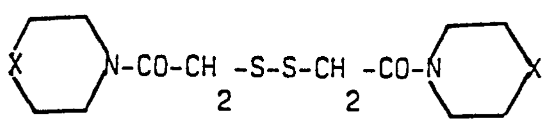

in der X eine direkte Bindung, Sauerstoff oder Schwefel, Methylen, Ethylen oder N-R ist, wobei R Wasserstoff oder niedriges Alkyl oder Hydroxyalkyl, Formyl, Methyl, Propionyl oder Phenyl ist, wobei "niedriges Alkyl" Methyl, Ethyl, Propyl oder Isopropyl bedeutet, oder eines seiner gegebenenfalls pharmazeutisch verträglichen Salze, gekennzeichnet durch
1) Behandlung eines niedrigen Alkyldithiodiacetats oder -mercaptoacetats mit einem sekundären zyklischen Amin der Formel

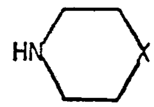

in der X wie oben definiert ist, bei einer Temperatur von 15 bis 60° C,

2) Oxidation des erhaltenen Produkts, wenn die Ausgangsverbindung ein niedriges Alkylmercaptoacetat ist,

3) gegebenenfalls Umwandlung des in einer der Stufen 1) oder 2) erhaltenen Produkts in eines seiner pharmazeutisch verträglichen Salze,

(der Ausdruck "niedriges Alkyl" bedeutet hier Methyl, Ethyl, Propyl und Isopropyl).

2. Verfahren nach Anspruch 1,
gekennzeichnet durch
Verwendung eines Lösungsmittels in Stufe 1).

3. Verfahren nach Anspruch 2,
gekennzeichnet durch
Verwendung eines Alkohols als Lösungsmittel.

4. Verfahren nach einem der Ansprüche 1 bis 3,
gekennzeichnet durch
Verwendung eines niedrigen Alkylmercaptoacetats als Ausgangsprodukt, das mit einem zyklischen sekundären Amin der Formel II

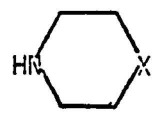

in der X wie in Anspruch 1 definiert ist, zu einer Verbindung der Formel III umgesetzt wird,

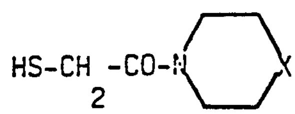

in der X wie oben definiert ist,
Oxidation der Verbindung der Formel III mit Luft oder in Gegenwart eines Oxidationsmittels, wie Brom, Jod oder sauerstoffhaltigen Wassers zu der Verbindung der Formel I, wobei der Ausdruck "niedriges Alkyl" Methyl, Ethyl, Propyl und Isopropyl bedeutet.

5. Verfahren nach Anspruch 5,
gekennzeichnet durch
Verwendung einer wäßrigen Lösung mit 15 % Sauerstoff als Oxidationsmittel bei einer Temperatur von 10 bis 30° C.

**Claims** for the contracting states, BE, CH, DE, FR, GB, IT, LI, LU, NL, SE.

1. Cyclic dithiodiacetamide of formula:

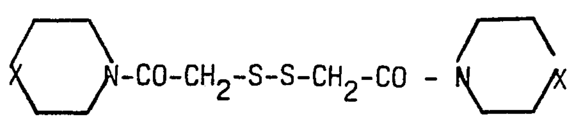

in which X represents a direct bond, an atom of oxygen or of sulfur, a methylene group, an ethylene group or a N-R group, where R is an atom of hydrogen or a lower alkyl group, lower (hydroxy) alkyl group, a formyl, acetyl, propionyl group, or a phenyl group, or one of its possible pharmaceutically acceptable salts, the term lower alkyl designating the methyl, ethyl, propyl and isopropyl groups.

2. 4,4'-dithiodiacetyldimorpholine.

3. 1,1'-dithiodiacetyldipyrrolidine.

4. 4,4'-dithiodiacetyldi-(1-methyl)piperazine.

5. Process for the preparation of a compound according to claim 1, characterized in that a dithiodiacetate or a mercaptoacetate of lower alkyl is treated with an amine of formula

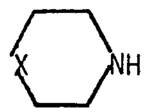

in which X is as defined in claim 1, at a temperature of between 15 and 60°C, and when a lower alkyl meracaptoacetate is used as starting product, the product so obtained is subjected to oxidation; and the product obtained is possibly converted into one of its pharmaceutically acceptable salts, the term lower alkyl designating the methyl, ethyl, propyl and isopropyl groups.

6. Pharmaceutical composition for inhibiting the malformation of the destruction of the red blood corpuscles due to a genetic modification of the haemoglobin or to parasites, characterized in that it contains, as active principle, a compound according to any one of claims 1 to 4.

7. Composition according to claim 6, characterized in that it is in dosage unit form.

8. Composition according to claim 7, characterized in that each dosage unit contains from 10 to 100 mg of active principle.

**Claims** for the contracting state : AT

1. Process for producing cyclic dithiodiacetamide of formula:

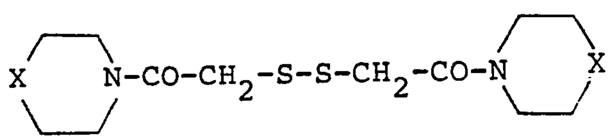

in which X represents a direct bond, an atom of oxygen or of sulfur, a methylene group, an ethylene group or a N-R group, where R is an atom of hydrogen or a lower alkyl group, lower (hydroxy)alkyl group, a formyl, acetyl , propionyl group, or a phenyl group, or one of its possible pharmaceutically acceptable salts, characterized in that it consists :

- in treating a dithiodiacetate or a mercaptoacetate of lower alkyl with a cyclic secondary amine of formula:

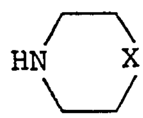

in which X is as defined above, at a temperature of between 15 and 60°C,

- in subjecting the product obtained to an oxidation when a lower alkyl mercaptoacetate is used as starting product,

- and possibly converting the product obtained in one of steps 1) or 2) into one of its pharmaceutically acceptable salts, the term lower alkyl designating the methyl, ethyl, propyl and isopropyl groups.

2. Process according to claim 1, characterized in that step 1) is carried out in a solvent.

3. Process according to claim 2, characterized in that the solvent is an alcohol.

4. Process according to any one of claims 1 to 3, characterized in that the starting product is a lower alkyl mercaptoacetate which gives by reaction with a secondary cyclic amine of formula II:

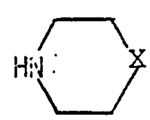

in which X is as defined in claim 1, a compound of formula:

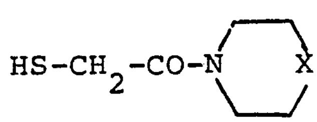

in which X is as defined above, said compound of formula III is then subjected to air oxidation or in the presence of an oxidizing agent, such as bromine, iodine, or peroxide of hydrogen to give a compound of formula I, the term lower alkyl designating the methyl, ethyl, propyl and isopropyl groups.

5. Process according to claim 4, characterized in that a 15 % solution of peroxide of hydrogen is used as oxidizing agent at the temperature of 10 to 30°C.